# EUROPEAN PATENT APPLICATION

(11) **EP 4 566 451 A1**
(43) Date of publication of application: **11.06.2025**
(21) Application number: 23849839.8
(22) Date of filing: 10.07.2023
(51) Int. Cl.: A01P 3/00, F24F 6/12, A61L 9/14, A01N 59/08, F24F 8/24, F24F 8/28, F24F 8/80

(54) **DISINFECTION OR INACTIVATION METHOD FOR SPACE**

(30) Priority: 05.08.2022 JP 2022125525
(71) Applicant: Nipro Corporation, Osaka 566-8510 (JP); Health Support Sanri Corporation, Osaka-shi, Osaka 532-0011 (JP)
(72) Inventor: SANO, Yoshihiko, Settsu-shi, Osaka 566-8510 (JP); SHIOYAMA, Tadao, Osaka-shi, Osaka 532-0011 (JP)
(74) Representative: M. Zardi & Co S.A.
(86) International application number: PCT/JP2023/025449
(87) International publication number: WO 2024/029280

(57) **Abstract**

Provided is a method of disinfecting or inactivating a germ or virus floating in a space, the method comprising the steps of: preparing a humidifier filled with an aqueous solution containing hypochlorous acid as a main component; and operating the humidifier to spray the aqueous solution containing hypochlorous acid as a main component into the space; wherein the spraying is performed while humidifying the space, and wherein 40 mol% or more of chlorine in the aqueous solution is chlorine that makes up hypochlorous acid.

## Description

### TECHNICAL FIELD

The present invention relates to a method of disinfecting or inactivating a germ or virus floating in a space by using an aqueous solution containing hypochlorous acid as a main component.

### BACKGROUND ART

In recent years, novel coronaviruses have been raging around the world, and the seventh wave of infection is about to hit Japan. The main route of infection for the novel coronaviruses is aerosol infection.

In response to this, various methods and systems for disinfecting or inactivating germs or viruses floating in spaces have begun to appear on the market. On the other hand, because the effectiveness of such methods and systems has not been sufficiently proven, the Consumer Affairs Agency has repeatedly issued corrective recommendations, claiming that those are recognized as "misleading representations" under the Act against Unjustifiable Premiums and Misleading Presentations.

Then, above all, there has been a demand for a method of disinfecting or inactivating floating germs or viruses that is safe for a human body.

The present inventors have conducted intensive studies in which using an aqueous solution containing hypochlorous acid as a main component that satisfies certain conditions is an effective means for disinfecting or inactivating floating germs or viruses that is safe for a human body (see Patent Documents 1 and 2).

### PRIOR ART DOCUMENT

### Patent Document

Patent Document 1: JP 2021-171323 A
Patent Document 2: JP 2021-172649 A

### SUMMARY OF THE INVENTION

### PROBLEMS TO BE SOLVED BY THE INVENTION

Furthermore, when this aqueous solution is used to disinfect or inactivate germs or viruses floating in a space, it is desirable that it can be used universally in general households and facilities without requiring a dedicated spray device or special method.

On the other hand, commercially available humidifiers mainly come in a heating (steam) type, an evaporative type, an ultrasonic type, and a hybrid type that combines both the heating type and the ultrasonic type. In particular, when an ultrasonic type humidifier is used to humidify a space with tap water, there is a problem that Legionella bacterium (hereinafter referred to as Legionella) proliferate in a tank and a spray mechanism inside a humidifier that has been used for a long period of time without internal cleaning and are dispersed into the space, causing Legionnaires' disease. Legionnaires' disease is a respiratory infection caused primarily by ingestion of Legionella that inhabits widely in the environment with aerosol into lungs. Legionella inhabits in the natural environments such as soil and river, but their bacterial number in the natural environment is not high. It is considered that the main cause of Legionnaires' disease is Legionella that occurs and proliferates in artificially created water environments.

Thus, it is an object of the present invention to provide a method of disinfecting or inactivating a germ or virus floating in a space, which can be used more efficiently and safely for a human body and can be further used for universal purposes in general households and facilities.

It is another object of the present invention to provide a method of using a humidifier that can suppress proliferation of Legionella in the humidifier.

### MEANS TO SOLVE THE PROBLEM

As a result of studies on the above-described problems, the present inventors have found that, by using an aqueous solution containing hypochlorous acid as a main component that satisfies certain conditions to spray it while humidifying with a humidifier, a germ or virus floating in a space can be disinfected or inactivated more efficiently while suppressing proliferation of Legionella in the humidifier, and completed the present invention. This method of the present invention involves spraying an aqueous solution with a low chlorine concentration into the space and is therefore safe for a human body.

That is, the present invention relates to:
[1] A method of disinfecting or inactivating a germ or virus floating in air, the method comprising the steps of:
   preparing a humidifier filled with an aqueous solution containing hypochlorous acid as a main component; and
   operating the humidifier to spray the aqueous solution into the space;
   wherein the spraying is performed while humidifying the space, and
   wherein 40 mol% or more, preferably 50 mol% or more, more preferably 60 mol% or more, further preferably 70 mol% or more, particularly preferably 80 mol% or more, and most preferably 90 mol% or more of chlorine in the aqueous solution is chlorine that makes up hypochlorous acid,
[2] The method of [1] above, wherein proliferation of Legionella bacterium in the humidifier is suppressed,
[3] The method of [1] or [2] above, wherein the humidifier is of an ultrasonic type,
[4] The method of any one of [1] to [3] above, wherein a sum of alkali metal ions and alkaline earth metal ions in the aqueous solution is less than 1.0 mEq/L, preferably 0.8 mEq/L or less, and further preferably 0.5 mEq/L or less,
[5] The method of any one of [1] to [4] above, wherein a total concentration of chlorine in the aqueous solution is 1.0 to 20.0 ppm,
[6] The method of [5], wherein the total concentration of chlorine in the aqueous solution is 1.0 to 5.0 ppm,
[7] The method of [6] above, wherein the total concentration of chlorine in the aqueous solution is 3.0 to 5.0 ppm,
[8] The method of any one of [1] to [7] above, wherein the spraying is performed while humidifying so that a relative humidity in the space is increased by 10 to 60% RH, preferably by 10 to 40% RH, and more preferably by 10 to 20% RH,
[9] The method of any one of [1] to [8] above, wherein the aqueous solution filled in the humidifier is replaced once a day, and
[10] A method of using a humidifier that suppresses proliferation of Legionella bacterium,
   wherein an aqueous solution containing hypochlorous acid as a main component, in which 40 mol% or more of chlorine in the aqueous solution is chlorine that makes up hypochlorous acid, is used for humidification.

### EFFECTS OF THE INVENTION

According to the present invention, a commercially available humidifier can be used, and by using an aqueous solution containing hypochlorous acid as a main component, in which 40 mol% or more of chlorine in the aqueous solution is chlorine that makes up hypochlorous acid, a method of disinfecting or inactivating a germ or virus floating in a space, which can be used more efficiently and safely for a human body and can be further used for universal purposes in general households and facilities, can be provided.

Furthermore, according to the method of using the humidifier of the present invention, proliferation of Legionella in the humidifier can be suppressed.

### BRIEF DESCRIPTION OF THE DRAWINGS

FIG. 1 is a diagram showing results of a test of suppressing proliferation of Legionella in an ultrasonic bath of an ultrasonic type humidifier.
FIG. 2 is a diagram explaining an arrangement of equipment in a test space for a spatial disinfection test.
FIG. 3 is a graph showing a disinfection rate (%) of Staphylococcus aureus in the spatial disinfection test.
FIG. 4 is a graph showing an inactivation rate (%) of Escherichia coli phage MS2 in a spatial inactivation test.
FIG. 5 is a graph showing an inactivation rate (%) of Escherichia coli phage MS2 in a spatial inactivation test.
FIG. 6 is a graph showing an inactivation rate (%) of Escherichia coli phage MS2 in a spatial inactivation test.
FIG. 7 is a graph showing an inactivation rate (%) of Escherichia coli phage MS2 in a spatial inactivation test.
FIG. 8 is a graph showing an inactivation rate (%) of Escherichia coli phage MS2 in a spatial inactivation test.
FIG. 9 is a graph showing an inactivation rate (%) of Escherichia coli phage MS2 in a spatial inactivation test.

### EMBODIMENT FOR CARRYING OUT THE INVENTION

As mentioned above, one aspect of the present invention is a method of disinfecting or inactivating a germ or virus floating in a space (hereinafter also simply referred to as a spatial disinfection), the method comprising the steps of: preparing a humidifier filled with an aqueous solution containing hypochlorous acid as a main component; and operating the humidifier to spray the aqueous solution containing hypochlorous acid as a main component into the space; wherein the spraying is performed while humidifying the space, and wherein 40 mol% or more of chlorine in the aqueous solution is chlorine that makes up hypochlorous acid. Although we tested for any effect on disinfecting or inactivating germs or viruses floating in the space when actually operating the humidifier with simple water (purified water) for humidification, we could not observe any effect. On the other hand, it has been observed that, by humidifying with an aqueous solution containing hypochlorous acid as a main component, in which 40 mol% or more of chlorine in the aqueous solution is chlorine that makes up hypochlorous acid, in accordance with the method of the present invention, the germ or virus floating in the space can be disinfected or inactivated more efficiently.

The Germ to be subjected to the spatial disinfection is not particularly limited as long as they float in a space, example of which include, for example,
- germs belonging to gram-negative facultative anaerobic rod-sharped bacteria such as the genera Escherichia, Shigella, Salmonella, Klebsiella, Proteus, Yersinia, Vibrio, Pasteurella, Campylobacter, Helicobacter, Yersinia, Serratia, Borrelia, Treponema, Flavobacterium, Capnocytophaga, and Haemophilus,
- germs belonging to gram-negative aerobic rod-sharped bacteria such as the genera Pseudomonas, Bordetella, Brucella, Bartonella, Aeromonas, Burkholderia, Acinetobacter, Stenotrophomonas, and Francisella,
- germs belonging to gram-negative obligate anaerobic rod-sharped bacteria such as the genera Bacteroides and Porphyromonas,
- germs belonging to gram-negative cocci such as the genera Moraxella and Neisseria,
- germs belonging to gram-positive cocci, such as the genera Staphylococcus, Streptococcus, Enterococcus, Mycobacterium, and Micrococcus,
- germs belonging to gram-positive rod-sharped bacteria such as the genera Clostridioides, Bacillus, Corynebacterium, Listeria, Nocardia, Propionibacterium, Bifidobacterium, Lactobacillus, Actinomyces, and Eubacterium,
- germs belonging to atypical bacteria such as the genera Mycoplasma, Legionella, Chlamydia, Chlamydophila, Rickettsia, Ehrlichia, Anaplasma, Neorickettsia, and Coxiella,
- germs belonging to fungi such as the genera Candida, Aspergillus, Cryptococcus, Pneumocystis, Trichophyton, Microsporum, and Epidermophyton,
and the like.

The Virus to be subjected to the spatial disinfection is not particularly limited as long as they can float in a space, examples of which include, for example, a molluscum contagiosum virus, a herpes simplex virus, a varicella-zoster virus, a rotavirus, a human papilloma virus, a poliovirus, a coxsackievirus, a rhinovirus, a rubella virus, a measles virus, an influenza virus, a mumps virus, a respiratory syncytial virus, a hepatitis virus, a human immunodeficiency virus (HIV), a coronavirus, a novel coronavirus, and the like.

### (Aqueous solution containing hypochlorous acid as main component)

According to one embodiment of the present invention, the aqueous solution containing hypochlorous acid as a main component is not particularly limited as long as 40 mol% or more of a sum of chlorine in the aqueous solution is chlorine that makes up hypochlorous acid, but it is desirable that the aqueous solution can be filled into a humidifier that will be mentioned later, which conforms to the aqueous solutions containing hypochlorous acid as a main component (Embodiments 1 and 2) in Patent Documents 1 and 2 (JP 2021-171323 A and JP 2021-172649 A). The first embodiment and the second embodiment of the aqueous solution containing hypochlorous acid as a main component will be described below. Any description that does not specify a particular embodiment shall apply to any general aqueous solution containing hypochlorous acid as a main component, which is used in any aspect of the present invention.

In the present specification, "ppm" (mg/kg) used as a unit of concentration is used in place of mg/L unless otherwise specified.

In the present specification, "containing hypochlorous acid as a main component" means containing hypochlorous acid as a representative component other than water as a solvent, and it does not mean that hypochlorous acid accounts for the majority of components and that hypochlorous acid is the most abundant component among components other than water. Therefore, as long as it does not adversely affect its disinfecting/sterilizing function or storage stability, other components may be contained besides water and hypochlorous acid as main components, particularly components containing chlorine and components that adjust pH, for example, HCl can be contained. On the other hand, in a conventional hypochlorous acid water, contents of undecomposed sodium chloride and hydrogen chloride that is a component contributing to pH are large, and a proportion of hypochlorous acid is low.

The aqueous solution containing hypochlorous acid as a main component used in the present invention is characterized in that 40 mol% or more of a sum of chlorine in the aqueous solution is chlorine that makes up hypochlorous acid. This indicates an amount of hypochlorous acid in the aqueous solution by a ratio of a molar amount of chlorine derived from hypochlorous acid, with a sum of molar equivalents of components containing chlorine atoms being 100 mol%, in the aqueous solution. As a result, even if the aqueous solution contains HCl, an amount of HCl does not exceed 60 mol% of the sum of chlorine in the aqueous solution, and the pH of the aqueous solution can be made closer to neutral as possible, so that it is considered that an aqueous solution containing hypochlorous acid as a main component, which is excellent in storage stability, can be obtained. Moreover, such an aqueous solution shall have less concern of causing corrosion of metal and deterioration of resin.

In the aqueous solution containing hypochlorous acid as a main component, 40 mol% or more of the sum of chlorine in the aqueous solution is chlorine that makes up hypochlorous acid, i.e., hypochlorous acid-derived chlorine, and preferably 50 mol% or more, more preferably 60 mol% or more, further preferably 70 mol% or more, particularly preferably 80 mol% or more, and most preferably 90 mol% or more of the sum of chlorine in the aqueous solution is hypochlorous acid-derived chlorine. If a total amount of chlorine in the aqueous solution is large, chlorine gas becomes easy to be generated. For example, in a case where the aqueous solution is electrolyzed water, when a ratio of hypochlorous acid-derived chlorine to the sum of chlorine in the aqueous solution is less than 40 mol%, a ratio of hydrogen chloride increases, and damages, such as irritation and corrosion, caused by hydrogen chloride tend to become large. Metal corrosion refers to metal ions bonding with chlorine, bases (hydroxyl group, nitrate group, sulfate group, and the like), oxygen, and the like, which occurs by the metal being deprived of electric charges therein, ionized, and fallen off.

A sum of concentration of chlorine in the aqueous solution containing hypochlorous acid as a main component is preferably 1.0 to 20.0 ppm. More preferably, it is 1.0 to 5.0 ppm, and further preferably, it is 3.0 to 5.0 ppm. The concentration may be adjusted by diluting with purified water or the like. Even at this extremely low concentration of chlorine, a sufficient spatial disinfection effect can be obtained, and it is considered that there is almost no influence on a human body moving around in a sprayed space (a concentration of chlorine considered to have no influence on human health in Guidelines for drinking-water quality by WHO is 5 mg/L or less, i.e., 5 ppm or less). Besides, when the aqueous solution containing hypochlorous acid as a main component used in the present invention is sprayed into a space, a concentration of chlorine in the space is in a range of approximately 0.005 to 0.01 ppm.

As water used for preparing an aqueous solution containing hypochlorous acid as a main component, pure water, purified water, ion-exchanged water, or the like is preferably used in order to suppress contamination of unplanned components such as, for example, metal ions and organic substances contained in tap water.

In the aqueous solution according to the first embodiment of the aqueous solution containing hypochlorous acid as a main component, it is preferable that the aqueous solution contains as few alkali metal ions and alkaline earth metal ions as possible. Specifically, a sum of alkali metal ions and alkaline earth metal ions is preferably less than 1.0 mEq/L, more preferably 0.8 mEq/L or less, and further preferably 0.5 mEq/L or less. By setting the sum of alkali metal ions and alkaline earth metal ions to less than 1.0 mEq/L, generation of chlorine gas is suppressed, and stability tends to be increased. In other words, by making the aqueous solution contain as few alkali metal ions and alkaline earth metal ions, which are counter ions to chlorine, as possible, generation of hydrogen chloride gas and decomposition (inactivation) of hypochlorous acid can be suppressed. When such an aqueous solution is sprayed indoors, a concentration of hydrogen chloride and concentration of salt are low, therefore making it possible to suppress corrosion, particularly influence on an electronic substrate or the like.

The alkali metal ions and alkaline earth metal ions to be contained for adjusting pH when necessary are not particularly limited, and each can be added in a form of a salt. Examples of such a salt of an alkali metal ion include a salt of sodium (Na) or potassium (K), and examples of such a salt of an alkaline earth metal ion include a salt of magnesium (Mg) or calcium (Ca). On the other hand, it is preferable not to use salts of rubidium (Rb) and osmium (Os) since they are known to be carcinogenic.

In the aqueous solution according to the first embodiment of the aqueous solution containing hypochlorous acid as a main component, for anions in the aqueous solution, a concentration of chloride other than hypochlorous acid is preferably less than 1.0 mmol/L, more preferably 0.9 mmol/L or less, and further preferably 0.4 mmol/L or less.

The aqueous solution according to the first embodiment of the aqueous solution containing hypochlorous acid as a main component is preferably an electrolysis water, specifically an electrolysis water containing hypochlorous acid as a main component, which is obtained by performing electrolysis that uses hydrochloric acid or sodium chloride as a raw material.

The pH of the aqueous solution according to the first embodiment of the aqueous solution containing hypochlorous acid as a main component is preferably 2.7 or higher, more preferably 3.2 or higher, further preferably 3.5 or higher, and particularly preferably 5.8 or higher. When the pH of the aqueous solution containing hypochlorous acid as a main component is 2.7 or higher, corrosiveness is reduced, and the aqueous solution tends to be able to be used for various applications. In particular, when the aqueous solution containing hypochlorous acid as a main component is used for a humidifier, an air cleaner, or the like, there is a concern about corrosion and deterioration of parts used in a device, and therefore the pH is preferably 3.2 or higher. When the pH is 3.2 or higher, generation of chlorine gas (hydrogen chloride gas) can be further suppressed. In addition, it is preferable that the pH of the aqueous solution containing hypochlorous acid as a main component is 5.8 or more and 6.5 or less, which reduces irritation to a mucous membrane, etc. and at the same time meets standards for drinking water.

The aqueous solution according to the first embodiment of the aqueous solution containing hypochlorous acid as a main component can be used for various applications due to its not too low pH and strong bactericidal and antiviral properties, for example, it can be widely used in various fields such as public health for disinfection, sterile filtration, and the like in daily life, food, and medical care. It can be appropriately used for disinfection and bactericidal/sterile filtration of various items such as clothing, tableware, glass containers, and plastic containers and environments in households and medical settings, particularly for cleaning and disinfection of skins such as hand and finger washing (disinfection by hand washing) and spatial disinfection by adjusting the pH. When tap water is used for a humidifier, an air cleaner, or the like, germs such as mold may be generated in a water storage tank and the generated germs may be scattered, but on the other hand, the above-mentioned aqueous solution containing hypochlorous acid as a main component contains hypochlorous acid having a high disinfection effect, so that it can maintain the hygiene not only inside the device but also in a space, and therefore it is preferably used for a humidifier or an air cleaner instead of tap water. In particular, in an aspect that does not contain any salt and has a pH that is not too low, there is little concern about corrosion, which is also considered to be more preferable for use in a humidifier and an air cleaner.

The aqueous solution according to the first embodiment of the aqueous solution containing hypochlorous acid as a main component can be prepared by various methods. For example, first, in a two-diaphragm three-chamber type electrolytic cell, in which an anode chamber, a cathode chamber, and an intermediate chamber containing an electrolytic solution provided between the anode chamber and the cathode chamber are separated by diaphragms, respectively, electrolysis is performed using an aqueous solution of sodium chloride as an electrolytic solution to obtain an acidic hypochlorous acid water on the anode chamber side. At this time, generally, chloride and sodium migrate to the anode and the cathode, respectively, and the migrating chloride reacts with water to produce hydrogen chloride (HCl) and hypochlorous acid (HClO), while generating oxygen gas. On the cathode side, sodium reacts with water to produce sodium hydroxide, while generating hydrogen gas. For the reaction between sodium chloride and water, as shown in the following expression, a ratio of hypochlorous acid generated to hydrogen chloride generated is stoichiometrically not more than 1:1, and therefore a proportion of hypochlorous acid generated does not exceed that of hydrogen chloride generated:

2NaCl + 3H₂O → 2NaOH + HCl + HClO + H₂

Next, the obtained acidic hypochlorous acid water is electrolyzed again to convert hydrogen chloride into hypochlorous acid, and the proportion of hypochlorous acid to hydrochloric acid can be increased. By increasing the ratio of hypochlorous acid to hydrochloric acid, the pH can be sloped toward the neutral side at the same time. This treatment (electrolysis of the hypochlorous acid water) can be repeated as many times as necessary to obtain a desired ratio of hypochlorous acid to hydrochloric acid and a desired pH.

An aqueous solution according to the second embodiment of an aqueous solution containing hypochlorous acid as a main component is obtained by adding an alkaline substance to the aqueous solution according to the first embodiment to adjust the pH to 5.0 to 7.0. Since an acid dissociation constant of hypochlorous acid is 7.5, a proportion of undissociated hypochlorous acid in the aqueous solution exceeds 50% by setting the pH to 7.0 or less (a proportion of undissociated hypochlorous acid in the aqueous solution becomes 90% or more by setting the pH to 6.5 or less), so that a higher sterilizing performance can be exhibited. Moreover, the pH is set to 5.8 or more, which meets the standards for drinking water. In the second embodiment of the aqueous solution containing hypochlorous acid as a main component, the pH is more preferably 5.8 to 6.5. Here, as the alkaline substance, a variety of pH adjusting agents can be used, specifically sodium hydroxide, potassium hydroxide, calcium hydroxide, sodium hydrogencarbonate, potassium hydrogencarbonate, calcium hydrogencarbonate, magnesium hydrogencarbonate, sodium acetate, potassium acetate, calcium acetate, magnesium acetate, sodium citrate, potassium citrate, calcium citrate, or magnesium citrate, and an aqueous solution thereof. Among them, sodium hydroxide and an aqueous solution thereof are particularly preferable.

The aqueous solution according to the second embodiment of the aqueous solution containing hypochlorous acid as a main component has a pH of 5.0 to 7.0, which is neutral to weakly acidic, so that the pH becomes close to a pH of a skin, greatly reducing occurrence of irritation due to acid and of itching due to alkali. Moreover, generation of trihalomethanes can be suppressed by not tilting the pH toward the alkali side. When the aqueous solution according to the second embodiment is sprayed indoors, a concentration of hydrogen chloride and concentration of salt are low, therefore make it possible to suppress corrosion, particularly influence on an electronic substrate or the like. In addition to the application of the aqueous solution according to the first embodiment, the aqueous solution can be further used for various applications, for example, it can be appropriately used in particular for cleaning and disinfection of skins such as hand and finger washing (disinfection by hand washing), spatial disinfection, disinfection of an instrument such as, for example, a medical instrument, further disinfection of bedsore, and the like.

### (Humidifier)

The method of the present invention includes a system of spraying the above-mentioned aqueous solution containing hypochlorous acid as a main component into a space using a humidifier. This spraying system is not particularly limited as long as it converts the aqueous solution containing hypochlorous acid as a main component into fine particles, atomizes them, and then sprays them into the space. Moreover, according to the present invention, disinfection or inactivation of germs or viruses floating in the space can be achieved extremely efficiently by simultaneously humidifying the space. The humidifier used in the present invention may be a commercially available one, and any of a heating type, an evaporative (steam) type, an ultrasonic type, and a hybrid type that combines both the heating type and the ultrasonic type can also be used, but the ultrasonic type is preferable from the viewpoint of being able to stably spray the aqueous solution containing hypochlorous acid as a main component. Examples of the ultrasonic type humidifier used in the present invention include, for example, CL Mist L (manufactured by NIPRO CORPORATION), Ururi/Urure series (manufactured by NITORI Co., Ltd.), HM-201 (manufactured by SEIKO GIKEN INC.), and the like.

However, the ultrasonic type humidifier in particular has a problem of Legionella proliferating when using tap water, and hygiene must be maintained on a daily basis. However, the proliferation of Legionella in the humidifier can be suppressed by using the aqueous solution containing hypochlorous acid as a main component as mentioned above. For more hygienic purposes, it is more preferable to replace the aqueous solution containing hypochlorous acid as the main component, in which 40 mol% or more of chlorine in the aqueous solution is chlorine that makes up hypochlorous acid, once a day. As such, another aspect of the present invention is a method of using a humidifier to suppress proliferation of Legionella bacterium, characterized in that the aqueous solution containing hypochlorous acid as a main component, in which 40 mol% or more of chlorine in the aqueous solution is chlorine that makes up hypochlorous acid, is used for humidification. Moreover, a person skilled in the art will understand that the description of the method of disinfecting or inactivating the space according to the present invention also applies to the method of using the humidifier to suppress proliferation of Legionella in the humidifier, as long as there is no contradiction.

In the present invention, the above-mentioned aqueous solution containing hypochlorous acid as a main component, in which 40 mol% or more of chlorine in the aqueous solution is chlorine that makes up hypochlorous acid, is sprayed while humidifying the space. In one embodiment, desirably for a degree of humidification, the aqueous solution is preferably sprayed while humidifying so that a relative humidity of the space is increased by 10 to 60% RH, more preferably by 10 to 40% RH, and further preferably by 10 to 20% RH. Surprisingly, it has been found that disinfection or inactivation of germs or viruses floating in a space is achieved with a synergistic effect by simultaneously spraying into the space the aqueous solution containing hypochlorous acid as a main component, in which 40 mol% or more of chlorine in the aqueous solution is chlorine that makes up hypochlorous acid, and humidifying the space, according to the present invention. Since a humidifier generally has a spraying speed setting function in several stages, humidification is performed so as to reach an intended humidity with a hygrometer, installed in a space where a disinfection effect is to be expected, being as an indicator, using this setting. A time required for humidification to reach the intended relative humidity is not particularly limited because it is best to obtain a disinfection effect as soon as possible, but it is preferably within 60 minutes, and more preferably within 30 minutes. Furthermore, it becomes possible to disinfect or inactivate the space more effectively by replacing once a day the aqueous solution containing hypochlorous acid as a main component, in which 40 mol% or more of chlorine in the aqueous solution filled in the humidifier is chlorine that makes up hypochlorous acid, which is further preferable.

Although the present invention will be specifically described below based on Examples, it is not intended to be limited to these Examples.

### EXAMPLES

### Production example 1: Preparation of aqueous solution containing hypochlorous acid as main component (concentration: 10.0 ppm)

An aqueous solution of sodium chloride was electrolyzed as an electrolytic solution in a 2-diaphragm 3-chamber type electrolytic cell to obtain a strongly acidic hypochlorous acid water from the anode side. The obtained strongly acidic hypochlorous acid water was electrolyzed again to convert hydrogen chloride into hypochlorous acid to obtain an aqueous solution containing hypochlorous acid as a main component with an increased proportion of hypochlorous acid to hydrochloric acid. Sodium hydroxide was added to 1 L of the obtained aqueous solution containing hypochlorous acid as a main component to adjust the pH to 6.5. The pH was measured with a multiple water quality meter (MM-60, manufactured by Toa DKK-TOA CORPORATION). Furthermore, after adjusting the pH, an amount of chlorides other than hypochlorous acid was reduced by filtration process through a filtration membrane. Then, a concentration of a sum of chlorine was adjusted to 10.0 ppm by dilution with purified water. At that time, by confirming that the pH was within a range of 5.0 to 7.0, it was ensured that 40 mol% or more of chlorine in the aqueous solution was chlorine that makes up hypochlorous acid. Moreover, a concentration of a sum of alkali metal ions and alkaline earth metal ions in the aqueous solution was 0.8 mEq/L.

### Production example 2: Preparation of aqueous solution containing hypochlorous acid as main component (1.0 ppm)

The same procedure as in Production example 1 was performed except that a concentration of a sum of chlorine was adjusted to 1.0 ppm.

### Production example 3: Preparation of aqueous solution containing hypochlorous acid as main component (0.1 ppm)

The same procedure as in Production example 1 was performed except that a concentration of a sum of chlorine was adjusted to 0.1 ppm.

### Production example 4: Preparation of aqueous solution containing hypochlorous acid as main component (3.0 ppm)

The same procedure as in Production example 1 was performed except that a concentration of a sum of chlorine was adjusted to 3.0 ppm.

### Reference example 1

A disinfection effect of Legionella bacterium was confirmed using the following method. Legionella (Legionella pneumophila available from ATCC) was cultured in BCYEα medium (manufactured by Nissui Pharmaceutical Co., Ltd.) under a condition at 36±2°C for 18 to 24 hours, subcultured once, and then cultured again under the condition at 36±2°C for 18 to 24 hours. Bacteria were eradicated from colonies obtained by the culturing and then suspended in saline to prepare a bacterial suspension (suspension concentration: about 10⁸ CFU/mL). This suspension was mixed with the aqueous solution of Preparation example 1 for 5 minutes (volume ratio of 1:98), and then a reaction stop solution (0.5% sodium thiosulfate phosphate buffer) was added to the mixture (hereinafter, "treated group"). Moreover, one obtained by mixing the suspension and water for injection (volume ratio of 1:98) was used as a control (hereinafter, "control group"). Each of the treated group and the control group was cultured in BCYEα medium under the condition at 36±2°C for one day. The number of bacteria was assessed by counting the colonies obtained through the cultivation using a colony counter, and a disinfection effect was evaluated by calculating the reduction in the number of bacterial compared to the number of bacterial in the control group.

### Reference example 2

The same procedure as in Reference example 1 was performed except that the aqueous solution of Production example 2 was used instead of the aqueous solution of Production example 1.

### Reference example 3

The same procedure as in Reference example 1 was performed except that the aqueous solution of Production example 3 was used instead of the aqueous solution of Production example 1.

### Reference comparative example 1

The same procedure as in Reference example 1 was performed except that tap water was used instead of the aqueous solution of Production example 1. Besides, a concentration of chlorine in tap water was confirmed to be 0.16 ppm.

Results of Reference examples 1 to 3 and Reference comparative example 1 are shown in Table 1. Reference examples 1 to 3, in which the aqueous solutions of Production examples 1 to 3 were used, showed a high disinfection effect of 89% or more, and further, Reference examples 1 and 2, in which Production examples 1 and 2 with high concentrations of hypochlorous acid were used, showed a disinfection effect of 99.9% or more.

**Table 1**

| | The number of bacteria (CFU/mL) | | Disinfection rate (%) |
|---|---|---|---|
| | Control group | Treated group | |
| Reference example 1 | 7.8×10⁵ | 1.3×10 | >99.9 |
| Reference example 2 | 7.8×10⁵ | 6.7×10 | >99.9 |
| Reference example 3 | 7.8×10⁵ | 8.4×10⁴ | 89.1 |
| Reference comparative example 1 | 7.8×10⁵ | 7.5×10⁵ | 3.8 |

### Example 1

A test for suppressing proliferation of Legionella in an ultrasonic type humidifier was performed using the following method. An ultrasonic type humidifier (Ururi S (Registered Trademark), manufactured by NIPRO CORPORATION), in which a tank and a main ultrasonic bath had been cleaned and sterilized in advance, was prepared. 1.5 L of the aqueous solution of Production example 4 was filled in the tank of this humidifier. The humidifier filled with the aqueous solution of Production example 4 was then installed in a safety cabinet and operated for one hour per day to spray the aqueous solution of Production example 4. Besides, the humidifier was implemented with a setting of "medium" (about 110 mL/h ± 20%; catalog value).

The aqueous solution in the ultrasonic bath of the humidifier after spraying was sampled, and this sampled solution was cultured in BCYEα medium under a condition at 36±2°C for 3 days. A state of colonies formed after culture was photographed and confirmed.

The above-described operations were performed for a maximum of seven days. Besides, when colony formation was clearly confirmed, the test was discontinued on that day. Moreover, the aqueous solution in the tank of the humidifier was not replaced after completion of spraying on each day.

### Example 2

The same procedure as in Example 1 was performed except that the aqueous solution in the tank of the humidifier was replaced after completion of spraying on each day.

### Comparative example 1

The same procedure as in Example 1 was performed except that tap water was used instead of the aqueous solution of Production example 4.

### Comparative example 2

The same procedure as in Example 2 was performed except that tap water was used instead of the aqueous solution of Production example 4.

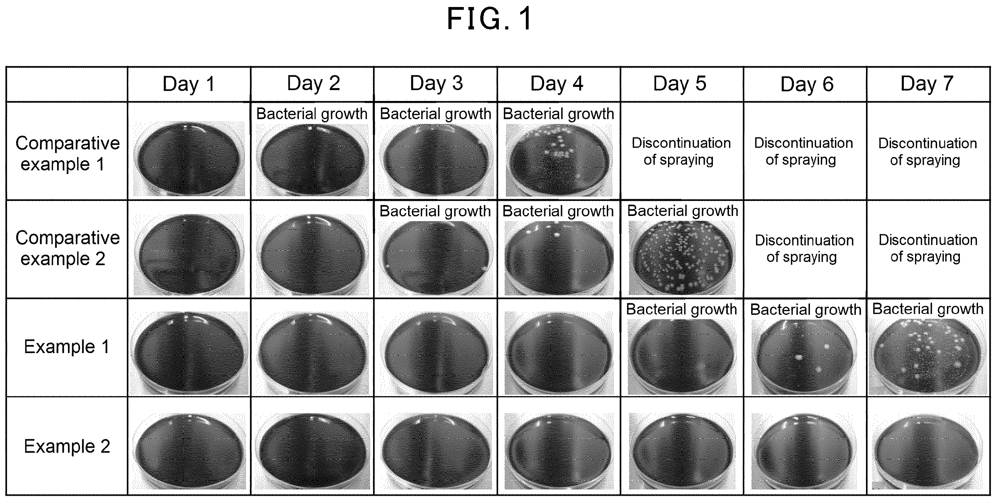

Results from Examples 1 and 2 and Comparative examples 1 and 2 are shown in FIG. 1. It was revealed that the aqueous solution of the present invention delayed the time of occurrence of bacteria in the humidifier compared to tap water, and that daily water changes could completely suppress bacterial growth during the test period.

### Example 3

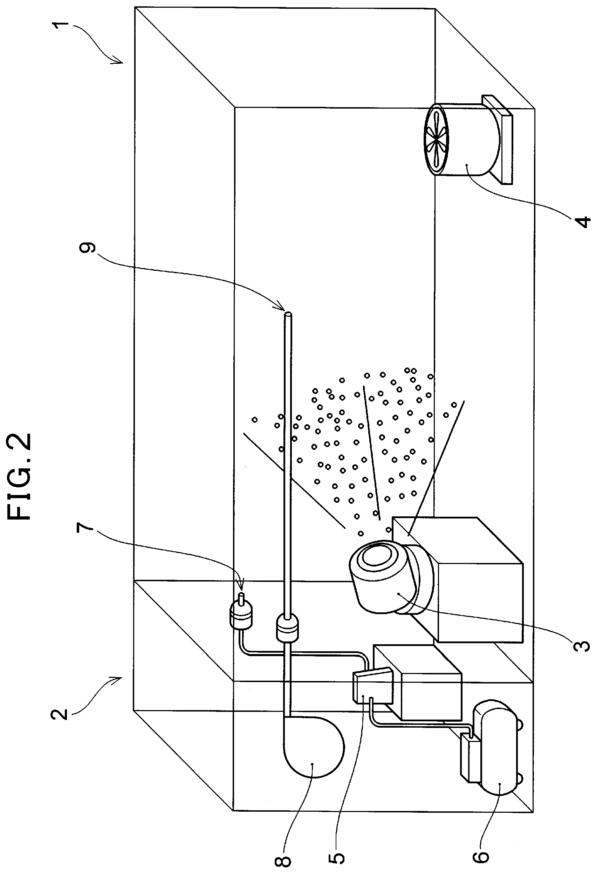

A spatial disinfection test for Staphylococcus aureus was performed using the following method. An arrangement of equipment in the experimentation is shown in FIG. 2.

In a 25.7 m³ (W: 2.7 m × D: 3.8 m × H: 2.4 m) of a test space 1, an ultrasonic type humidifier (CL Mist L (Registered Trademark) manufactured by NIPRO CORPORATIO) 3 was installed at the position shown in FIG. 2. The humidifier 3 was pre-filled with 3 L of the aqueous solution containing hypochlorous acid as a main component, prepared in Production example 4. A relative humidity in the space before operation of the humidifier 3 was set to 50% RH. In addition, a circulator 4 was installed in advance at the position shown in FIG. 2 and operated at a wind speed of 2 m/s, so that a sprayed aqueous solution containing hypochlorous acid as its main component and Staphylococcus aureus, which will be mentioned later, were sufficiently distributed in the test space 1.

Two mL of a separately prepared suspension of Staphylococcus aureus (about 10⁹ CFU/mL) was sprayed via a spray port 7 from an anterior room 2 using a nebulizer (Collison Nebulizer CN-31I manufactured by BGI Inc.) 5. After Staphylococcus aureus was purchased (purchased from National Institute of Technology and Evaluation), it was cultured in TSA medium (manufactured by Difco Laboratories) under a condition at 36±2°C for 1-2 days, then subcultured once, and cultured again under the condition at 36±2°C for 1 day. Bacteria were eradicated from colonies obtained through the cultivation and then suspended in saline to prepare the suspension. Besides, a concentration of the suspension is a value calculated from the number of colonies after sampling the obtained suspension and culturing it again in TSA medium (manufactured by Difco Laboratories) for one day.

For the purpose of uniforming the indoor air in the test space 1, the room was left to stand for 2 minutes after spraying the Staphylococcus aureus, and then the aqueous solution containing hypochlorous acid as a main component was sprayed into the test space 1 at a spray rate of 400 mL/h for 30 minutes using the humidifier 3. At this time, the humidifier 3 was set to a turbo mode so that the relative humidity in the test space 1 reached 90% RH in 30 minutes.

The time at which the room was left to stand for 2 minutes after spraying of Staphylococcus aureus was defined as the start time, 0 minute, and based on that time, disinfection rates (%) after 30, 60, and 120 minutes were measured. The disinfection rate (%) was calculated by collecting an air sample via a sample collection port 9 from the test space 1 using an impinger 8 shown in FIG. 2 at each measurement time, performing a predetermined treatment, and then counting the number of colonies obtained though the cultivation using a colony counter, with a ratio when the number of bacteria at the start time of 0 minute was taken as 100%. Specifically, for the predetermined treatment, 20 L of air sample in the test space 1 was poured from outside the test space 1 (anterior room 2) through a tube directly connected to the impinger 8 via the sample collection port 9 into 20 mL of saline containing 0.015% of sodium thiosulfate (manufactured by FUJIFILM Wako Pure Chemical Corporation). 10-fold serial dilution series were prepared with saline based on the obtained liquid, and each was cultured in SCD medium under a condition at 36±2°C for one day, after which the number of obtained colonies was counted using the colony counter.

In addition, 30 minutes after the start, a concentration of chlorine in the test space 1 was in a range of 0.005 to 0.01 ppm. The same applied to Examples 4 to 16 that will be mentioned later.

### Comparative example 3

The same procedure as in Example 3 was performed except that purified water was used instead of the aqueous solution containing hypochlorous acid as a main component prepared in Production example 4.

### Comparative example 4

The same procedure as in Example 3 was performed except that no humidification was performed using a humidifier. That is, the relative humidity in the space remains at 50%.

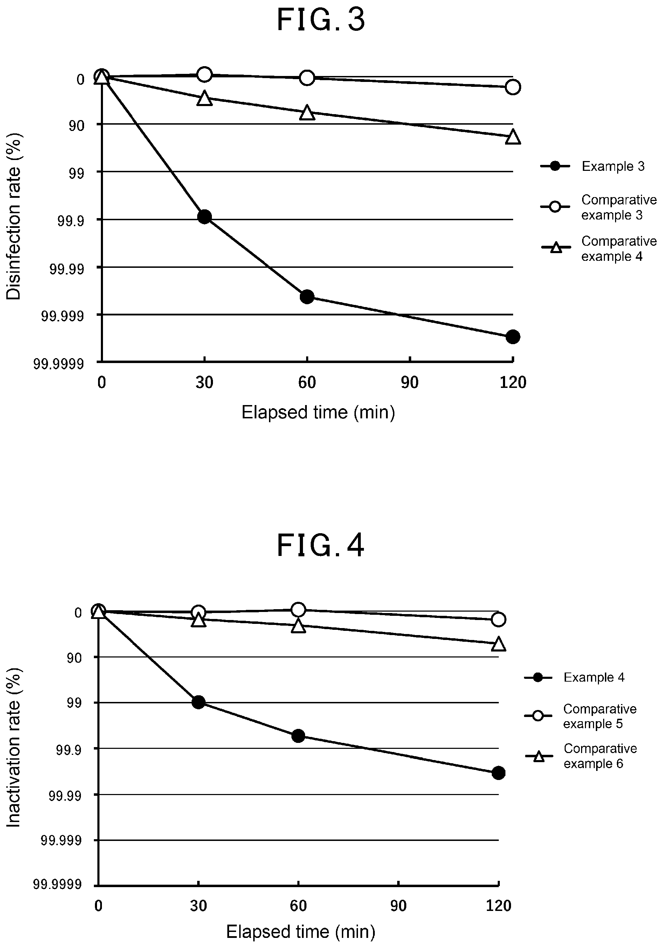

Results from Example 3 and Comparative examples 3 and 4 are shown in FIG. 3. **In** Example 3, a disinfection rate of 99.9% was already observed 30 minutes after the start time. **In** contrast, in Comparative example 4, a disinfection rate was 90% in 120 minutes. **In** Comparative example 3, no significant reduction in the number of bacteria was confirmed.

### Example 4

A spatial inactivation test against Escherichia coli phage MS2 was performed. A spraying operation of a virus suspension, spraying operation of a test sample, and a collecting operation of an air sample were performed in accordance with Example 3 except that Escherichia coli phage MS2 was used instead of Staphylococcus aureus. **In** addition, as a target was Escherichia coli phage MS2, a preparing operation of the virus suspension and a culturing operation of a collecting liquid that collected the air sample were switched to methods suitable for the corresponding virus. Additionally, a disinfection rate (%) shall be read as an inactivation rate (%).

A virus suspension was prepared specifically as follows. After Escherichia coli was purchased (purchased from National Institute of Technology and Evaluation), it was cultured in NB medium (manufactured by Difco Laboratories) under a condition at 36±2°C for 1-2 days, then subcultured once, and cultured again under the condition at 36±2°C for 1 day, followed by eradicated by the obtained colonies and suspended in saline. Thereto Escherichia coli phage MS2 (purchased from National Institute of Technology and Evaluation) was inoculated, mixed with a semisolid agar (NB medium (manufactured by Difco Laboratories) + 0.5% sodium chloride (manufactured by FUJIFILM Wako Pure Chemical Corporation) + 0.5% agar (manufactured by FUJIFILM Wako Pure Chemical Corporation)), and then layered on top of a normal agar medium (manufactured by Nissui Pharmaceutical Co., Ltd.). After culturing for 18 hours under a condition at 36±2°C, Escherichia coli was removed by centrifugation and filtered through a membrane filter having a pore size of 0.22 µm (manufactured by TPP Techno Plastic Products AG) to obtain a virus suspension mother liquid. This mother liquid was diluted 100-fold with a sterile ion-exchanged water to obtain a virus suspension (10⁹ PFU/mL) for testing. A concentration of the suspension is a value calculated from the number of plaques after sampling the obtained suspension, inoculating it again into Escherichia coli, and then culturing it.

A culturing operation of a collecting liquid that collected an air sample was performed specifically as follows. 10-fold dilution series were prepared with PBS (manufactured by ELMEX) based on the obtained liquid, and each was inoculated into an Escherichia coli suspension. An inoculum was mixed with a semisolid agar (NB medium (manufactured by Difco Laboratories) + 0.5% sodium chloride (manufactured by FUJIFILM Wako Pure Chemical Corporation) + 0.5% agar (manufactured by FUJIFILM Wako Pure Chemical Corporation)), and then layered on top of a normal agar medium (manufactured by Nissui Pharmaceutical Co., Ltd.), which was cultured under a condition at 36±2°C for 20 hours, and then the number of plaques obtained was counted.

### Comparative example 5

The same procedure as in Example 4 was performed except that purified water was used instead of the aqueous solution containing hypochlorous acid as a main component prepared in Production example 4.

### Comparative example 6

The same procedure as in Example 4 was performed except that no humidification was performed using a humidifier. That is, the relative humidity in the space remains at 50%.

Results from Example 4 and Comparative examples 5 and 6 are shown in FIG. 4. In Example 4, an inactivation rate of 99% was already observed 30 minutes after the start time. In contrast, in Comparative example 6, an inactivation rate was such an extent that it did not reach 90% even after 120 minutes. In Comparative example 5, no significant reduction in the number of viruses was confirmed.

### Example 5

A procedure was performed in accordance with Example 4 except that a relative humidity in a space before operation of a humidifier was set to 30%, and the humidifier was set so that the relative humidity in the space reached 40% RH in 30 minutes.

### Example 6

A procedure was performed in accordance with Example 4 except that a relative humidity in a space before operation of a humidifier was set to 30%, and the humidifier was set so that the relative humidity in the space reached 50% RH in 30 minutes.

### Example 7

A procedure was performed in accordance with Example 4 except that a relative humidity in a space before operation of a humidifier was set to 30%, and the humidifier was set so that the relative humidity in the space reached 60% RH in 30 minutes.

### Example 8

A procedure was performed in accordance with Example 4 except that a relative humidity in a space before operation of a humidifier was set to 40%, and the humidifier was set so that the relative humidity in the space reached 50% RH in 30 minutes.

### Example 9

A procedure was performed in accordance with Example 4 except that a relative humidity in a space before operation of a humidifier was set to 40%, and the humidifier was set so that the relative humidity in the space reached 60% RH in 30 minutes.

### Example 10

A procedure was performed in accordance with Example 4 except that a relative humidity in a space before operation of a humidifier was set to 50%, and the humidifier was set so that the relative humidity in the space reached 60% RH in 30 minutes.

### Example 11

A procedure was performed in accordance with Example 4 except that a relative humidity in a space before operation of a humidifier was set to 50%, and the humidifier was set so that the relative humidity in the space reached 70% RH in 30 minutes.

### Example 12

A procedure was performed in accordance with Example 4 except that a relative humidity in a space before operation of a humidifier was set to 50%, and the humidifier was set so that the relative humidity in the space reached 80% RH in 30 minutes.

### Example 13 (Example 4)

Conditions were the same as those in Example 4, but for ease of understanding, Example 13 was used.

### Example 14

A procedure was performed in accordance with Example 4 except that a relative humidity in a space before operation of a humidifier was set to 60%, and the humidifier was set so that the relative humidity in the space reached 70% RH in 30 minutes.

### Example 15

A procedure was performed in accordance with Example 4 except that a relative humidity in a space before operation of a humidifier was set to 60%, and the humidifier was set so that the relative humidity in the space reached 80% RH in 30 minutes.

### Example 16

A procedure was performed in accordance with Example 4 except that a relative humidity in a space before operation of a humidifier was set to 70%, and the humidifier was set so that the relative humidity in the space reached 80% RH in 30 minutes.

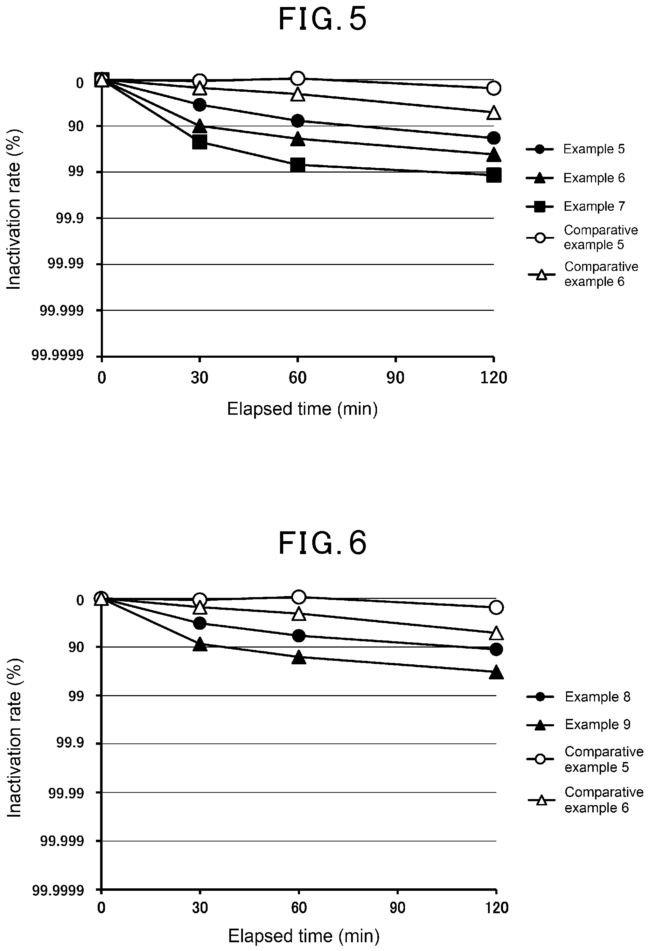

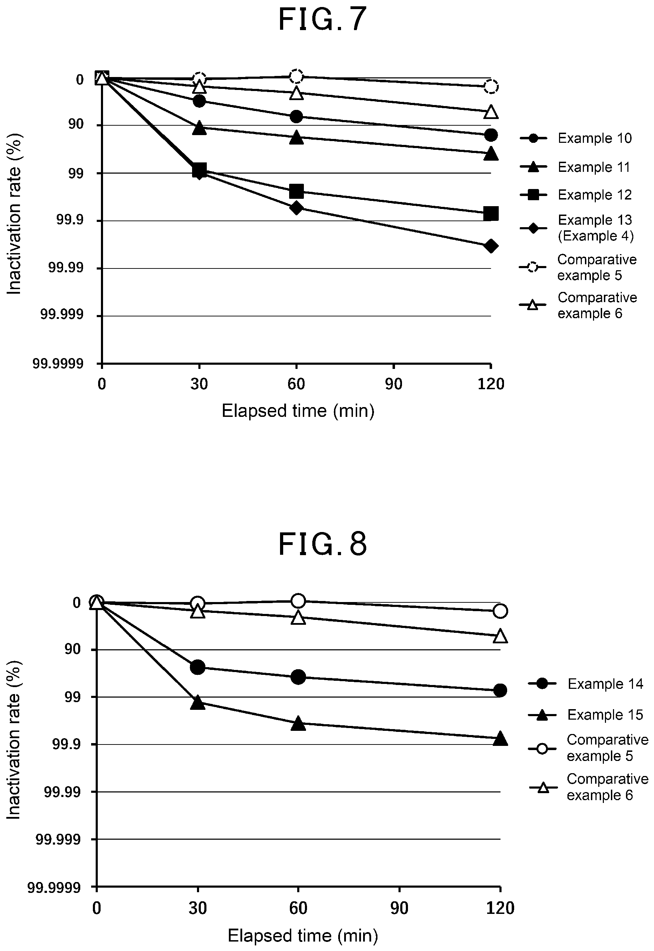

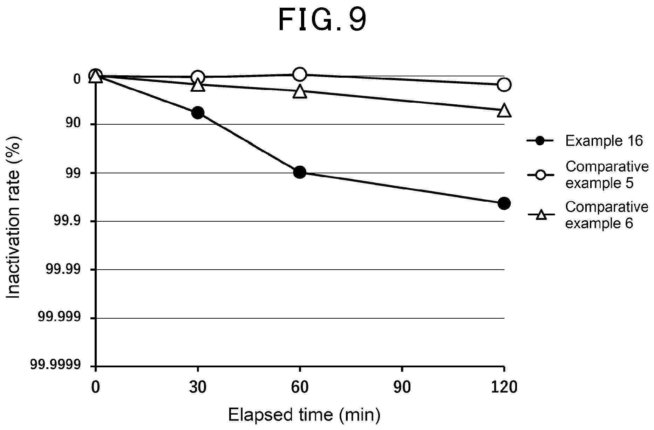

Results from Examples 5 to 7 are shown in FIG. 5, results from Examples 8 and 9 are shown in FIG. 6, results from Examples 10 to 13 are shown in FIG. 7, results from Examples 14 and 15 are shown in FIG. 8, and result from Example 16 are shown in FIG. 9, respectively. Besides, FIGS. 5 to 9 also show results from Comparative examples 5 and 6. Although the lower the relative humidity of the space becomes, the more difficult the inactivation becomes, even in Example 5, whose condition is considered to be most inferior (change in relative humidity: 30%→40%), it was possible to achieve 90% or more in between 60 and 120 minutes from the start time, and the result was better than that from Comparative example 6 (no humidification, relative humidity: maintained at 50%), and therefore it was shown that spraying the aqueous solution containing hypochlorous acid as a main component of the present invention while humidifying so as to increase the relative humidity of the space synergistically exhibited a disinfection or inactivation effect. Thus, according to the present invention, a sufficient disinfection or inactivation effect can be expected even during the winter season in Japan when humidity levels are particularly low.

In addition, in Examples of the present invention, Staphylococcus aureus was used as a representative model of germs. Germs (bacteria or fungi) have in common a basic structure of a cell membrane, a cell wall, and a nucleic acid, and hypochlorous acid achieve their disinfecting effects by denaturing membrane protein present in the cell membrane or the cell wall or by passing through the cell membrane and denaturing the nucleic acid within the cell, so that it is obvious that hypochlorous acid is also effective against germs other than Staphylococcus aureus.

Moreover, hypochlorous acid achieves its inactivating effect by denaturing an envelope or a membrane protein present in the envelope or by passing through the envelope and capsid and denaturing the nucleic acid therein. In Examples of the present invention, although Escherichia coli phage MS2 was used as a representative model of viruses, but Escherichia coli phage MS2 is a virus that does not possess an envelope. Nevertheless, a sufficient virus inactivation effect was confirmed in the present invention. That is, it can be scientifically considered that an even greater effectiveness can be expected against a novel coronavirus that possesses an enveloped.

### REFERENCE SIGNS LIST

1. Test space
2. Anterior room
3. Humidifier
4. Circulator
5. Nebulizer
6. Compressor
7. Spray port
8. Impinger
9. Sample collection port

## Claims

1. A method of disinfecting or inactivating a germ or virus floating in air, the method comprising the steps of:
preparing a humidifier filled with an aqueous solution containing hypochlorous acid as a main component; and
operating the humidifier to spray the aqueous solution into the space;
wherein the spraying is performed while humidifying the space, and
wherein 40 mol% or more of chlorine in the aqueous solution is chlorine that makes up hypochlorous acid.

2. The method of claim 1, wherein proliferation of Legionella bacterium in the humidifier is suppressed.

3. The method of claim 1 or 2, wherein the humidifier is of an ultrasonic type.

4. The method of claim 1 or 2, wherein a sum of alkali metal ions and alkaline earth metal ions in the aqueous solution is less than 1.0 mEq/L.

5. The method of claim 1 or 2, wherein a total concentration of chlorine in the aqueous solution is 1.0 to 20.0 ppm.

6. The method of claim 5, wherein the total concentration of chlorine in the aqueous solution is 1.0 to 5.0 ppm.

7. The method of claim 6, wherein the total concentration of chlorine in the aqueous solution is 3.0 to 5.0 ppm.

8. The method of claim 1 or 2, wherein the spraying is performed while humidifying so that a relative humidity in the space is increased by 10 to 60% RH.

9. The method of claim 1 or 2, wherein the aqueous solution filled in the humidifier is replaced once a day.

10. A method of using a humidifier that suppresses proliferation of Legionella bacterium,
wherein an aqueous solution containing hypochlorous acid as a main component, in which 40 mol% or more of chlorine in the aqueous solution is chlorine that makes up hypochlorous acid, is used for humidification.
